# EUROPÄISCHE PATENTSCHRIFT

(11) **EP 1 814 457 B1**
(45) Veröffentlichungstag und Bekanntmachung des Hinweises auf die Patenterteilung: **05.10.2011**
(21) Anmeldenummer: 05813666.4
(22) Anmeldetag: 21.11.2005
(51) Int. Cl.: A61B 6/00

(54) **STATIV FÜR EINE RÖNTGENANLAGE**
STAND FOR AN X-RAY SYSTEM
SUPPORT POUR UNE INSTALLATION DE RADIOGRAPHIE

(30) Priorität: 25.11.2004 DE 102004056995; 21.12.2004 DE 102004061506
(43) Veröffentlichungstag der Anmeldung: 08.08.2007
(73) Patentinhaber: SIEMENS AKTIENGESELLSCHAFT, 80333 München (DE)
(72) Erfinder: HESL, Stefan, 92676 Eschenbach (DE); HÄUPL, Rainer, 92703 Krummennaab (DE); WÖHRL, Dieter, 95478 Kemnath (DE)
(86) Internationale Anmeldenummer: PCT/EP2005/056122
(87) Internationale Veröffentlichungsnummer: WO 2006/056563

(56) Entgegenhaltungen:
- WO-A-80/01111
- GB-A- 1 175 032
- US-A- 3 373 285
- US-A- 3 803 418
- US-B1- 6 412 978

## Beschreibung

Die Erfindung bezieht sich auf ein Stativ für eine Röntgenanlage. Die Erfindung bezieht sich des Weiteren auf eine Röntgenanlage mit einem solchen Stativ.

Bei einer herkömmlichen Röntgenanlage, wie sie beispielsweise aus WO 80/01111 A1 bekannt ist, ist ein Stativ vorgesehen, das einen Röntgenstrahler und eine Kassettenlade haltert, wobei letztere zur Aufnahme einer Filmkassette, einer Speicherfolie oder dergleichen vorgesehen ist. Ein derartiges Stativ umfasst einen so genannten U-Bügel, der um eine Horizontalachse drehbar gelagert ist. Der U-Bügel trägt an einem ersten Schenkel den Röntgenstrahler und an einem zweiten Schenkel die Kassettenlade, so dass der Röntgenstrahler und die Kassettenlade unabhängig von der Schwenkstellung des U-Bügels im Raum stets in bezüglich einer Strahlrichtung zentrierter Gegenüberstellung fixiert sind. Die bekannte Röntgenanlage umfasst zwei Kassettenhalter. Beide Kassettenhalter sind um eine parallel zu der Horizonalachse angeordnete erste Kippachse sowie um eine zu dieser senkrecht angeordneten zweiten Kippachse schwenkbar.

Anstelle der herkömmlicherweise verwendeten Filmkassetten werden in jüngerer Zeit zunehmend digitale Röntgendetektoren eingesetzt, bei welchen ein aufgenommenes Röntgenbild in Form von digitalen Bilddaten erzeugt wird. Insbesondere sind zwischenzeitlich zweidimensionale Flachbilddetektoren kommerziell verfügbar, die mobil einsetzbar sind.

Aus US 6,412,978 B1 ist eine Röntgenanlage mit einem an einem U-Bügel fest montierten digitalen Röntgendetektor bekannt. Der Röntgendetektor ist über ein Kugelgelenk mit dem U-Bügel verbunden.

Stative für Röntgenanlagen sind ferner aus US 3,803,418 A, US 3,373,385 A sowie GB 1,175,032 A bekannt.

Der Erfindung liegt die Aufgabe zugrunde, ein Stativ für eine Röntgenanlage anzugeben, das zur Halterung eines digitalen Röntgendetektors besonders geeignet ist. Der Erfindung liegt weiterhin die Aufgabe zugrunde, eine geeignete, mit einem digitalen Röntgendetektor und einem derartigen Stativ versehene Röntgenanlage anzugeben.

Bezüglich des Stativs wird die Aufgabe erfindungsgemäß gelöst durch die Merkmale des Anspruchs 1. Danach umfasst das Stativ ein Sockelgestell, an welchem ein U-Bügel um eine Horizontalachse drehbar gelagert ist. Der U-Bügel umfasst einen ersten Schenkel zur Halterung eines Röntgenstrahlers sowie einen zweiten Schenkel, an welchem freiendseitig ein Detektorhalter angebracht ist. Der Detektorhalter umfasst wiederum ein Aufnahmeelement, in welchem ein insbesondere als Flachbilddetektor ausgebildeter digitaler Röntgendetektor in einer vorgegebenen Position verliersicher fixierbar ist. Der Detektorhalter ist hierbei derart ausgebildet, dass das Aufnahmeelement (gegebenenfalls zusammen mit dem darauf fixierten Röntgendetektor) sowohl um eine bezüglich der Horizontalachse parallel ausgerichtete Kippachse, als auch um eine hierzu senkrechte Drehachse verschwenkbar ist. Die Drehachse entspricht einer Flächennormalen auf eine durch die Erstreckungsrichtung des Aufnahmelements definierte Hauptebene, so dass diese Hauptebene gegenüber einer Verschwenkung des Aufnahmeelements um die Drehachse invariant ist. Hierdurch wird auch der auf dem Aufnahmeelement fixierte Röntgendetektor gedreht, ohne das seine im Wesentlichen zu der Hauptebene parallele Detektorfläche im Raum verkippt wird. Äquivalenterweise kann der Detektorhalter auch derart an dem U-Bügel befestigt sein, dass die Kippachse tangential, d.h. senkrecht und mit Abstand bezüglich der Horizontalachse ausgerichtet ist.

Durch die zweiachsige Verstellbarkeit des Detektorhalters ist der flexible Einsatz des Röntgendetektors für eine Vielzahl von unterschiedlichen Aufnahmepositionen ermöglicht. Vorteilhaft ist hierbei insbesondere die Möglichkeit, den Röntgendetektor um die Drehachse verdrehen zu können, zumal hierdurch die begrenzte Detektorfläche des digitalen Röntgendetektors im Hinblick auf die jeweilige Aufnahmeposition besonders gut ausnutzbar ist.

In bevorzugter Ausführung ist das Aufnahmeelement derart ausgebildet, dass der Röntgendetektor ohne Einsatz von Werkzeugen, und insbesondere einhändig fixierbar und wieder entnehmbar ist. Dies vereinfacht die Handhabung der Röntgenanlage in Hinblick auf einen mobilen Einsatz des Röntgendetektors. Insbesondere kann der Röntgendetektor hierdurch aus dem Detektorhalter des Stativs einfach entnommen und z.B. in ein separat stehendes Rasterwandgerät eingesetzt oder für eine freie Aufnahme genutzt werden.

Im Hinblick auf eine sichere und gleichzeitig einfach lösbare Befestigung des Röntgendetektors umfasst das Aufnahmeelement zweckmäßigerweise eine Anzahl von Klammerelementen, welche den Röntgendetektor in einer Fixierstellung allseitig umgreifen. In einer besonders vorteilhaften Ausführung ist ein solches Aufnahmeelement derart angeordnet, dass es eine Ecke des Röntgendetektors umgreift.

Für eine besonders einfache Befestigung bzw. Entnahme des Röntgendetektors ist vorzugsweise mindestens ein Klammerelement aus einer Fixierstellung elastisch auslenkbar. Alternativ hierzu ist eine Betätigungsmechanik vorgesehen, mittels welcher eines oder mehrere der Klammerelemente reversibel aus ihrer jeweiligen Fixierstellung auslenkbar sind.

In einer weiteren bevorzugten Ausführung bevorzugt das Aufnahmeelement eine Tasche, in welche der Röntgendetektor einschiebbar ist.

Der Detektorhalter umfasst zweckmäßigerweise weiterhin Rastmittel, mittels welcher das Aufnahmeelement bei einer Verschwenkung um die Kippachse und/oder bei einer Verschwenkung um die Drehachse in einer von mehreren Schwenkstellungen lösbar verrastet wird, um das Aufnahmeelement gegen eine ungewollte Selbstverdrehung zu sichern. Solche Rastmittel sind insbesondere nach Art einer Gefühlsraste ausgebildet, die bei Verschwenkung des Aufnahmeelements Druckpunkte erzeugt, in welchen das Aufnahmeelement einrastet, wobei diese Druckpunkte unter manueller Aufbringung eines entsprechenden Gegendruckes überwindbar sind. Alternativ ist das Aufnahmeelement durch einen manuell überwindbaren Reibschluss oder durch sonstige lösbare Feststellmechanismen gegen eine unbeabsichtigte Selbstverschwenkung gesichert.

Bei dem Stativ handelt es sich insbesondere um ein Deckenstativ. Das Sockelgestell ist entsprechend zur Befestigung an einer Raumdecke ausgebildet.

Bezüglich der Röntgenanlage wird die Aufgabe erfindungsgemäß gelöst durch die Merkmale des Anspruchs 9. Danach umfasst die Röntgenanlage einen digitalen Röntgendetektor sowie das zu dessen Halterung vorgesehene, vorstehend beschriebene Stativ. Die Röntgenanlage umfasst weiterhin eine Steuereinheit zur Ansteuerung, insbesondere zur Spannungsversorgung des Röntgendetektors. Die Steuereinheit ist hierbei an dem Sockelgestell des Stativs befestigt.

Durch den Einsatz des vorstehend beschriebenen Stativs wird, wie vorstehend beschrieben, eine flexible Verwendbarkeit des Röntgendetektors ermöglicht. Dies wird unterstützt durch die platzsparende und die Arbeitsvorgänge an der Röntgenanlage nicht behindernde Anbringung der Steuereinheit an dem Sockelgestell des Stativs.

Zweckmäßigerweise ist ein Versorgungskabel, das die Steuereinheit mit dem Röntgendetektor verbindet, in einer bezüglich des Stativs außenseitig angeordneten Kabelführung geführt. Dies ist insbesondere sinnvoll im mobilen Einsatz des Röntgendetektors, um einerseits bei am Stativ angebrachtem Röntgendetektor das Versorgungskabel wohlgeordnet am Stativ entlangführen zu können, und andererseits bei Verwendung desselben außerhalb des Stativs das Versorgungskabel leicht von dem Stativ entkoppeln zu können.

Die Kabelführung umfasst hierbei bevorzugt eine Rückholeinrichtung für das Versorgungskabel, die das Versorgungskabel automatisch in eine aus dem Arbeitsbereich der Röntgenanlage zurückgezogene Ruhelage zieht, sofern die volle Kabellänge nicht benötigt wird. Die Rückholeinrichtung ist insbesondere in Form einer selbst einziehenden Kabeltrommel und/oder eines an dem Versorgungskabel angreifenden Seilzugs realisiert. Zusätzlich oder alternativ umfasst die Kabelführung mindestens eine Punkthalterung, mittels welcher das Versorgungskabel an dem Stativ fixierbar ist. Um das Versorgungskabel dennoch für eine mobile Nutzung des Röntgendetektors flexibel handhaben zu können, ist die oder jede Punkthalterung derart ausgebildet, dass das Versorgungskabel aus der Fixierung werkzeugfrei lösbar ist. Die oder jede Punkthalterung ist bevorzugt in Form einer Magnethalterung ausgebildet. Alternativ hierzu ist vorgesehen, die Punkthalterung als Klemm- oder Klipsverbindung, Steckverbindung, Klettverschlussverbindung od.dgl. auszubilden.

Nachfolgend werden Ausführungsbeispiele der Erfindung anhand einer Zeichnung näher erläutert. Darin zeigen:
- FIG 1: in einer schematischen Seitenansicht eine Röntgenanlage mit einem Röntgenstrahler, einem digitalen Röntgendetektor sowie mit einem Stativ zur Halterung des Röntgenstrahlers und des Röntgendetektors,
- FIG 2: in perspektivischer Frontansicht vereinfacht die Röntgenanlage gemäß FIG 1,
- FIG 3: in perspektivischer Ansicht auf eine Oberseite einen Detektorhalter des Stativs sowie den auf einem Aufnahmeelement des Detektorhalters fixierten Röntgendetektor,
- FIG 4: in Darstellung gemäß FIG 3 den Detektorhalter in gegenüber FIG 3 um eine Kippachse und eine Drehachse verschwenkter Stellung des Aufnahmeelements,
- FIG 5: in perspektivischer Ansicht auf eine Rückseite den Detektorhalter gemäß FIG 4,
- FIG 6: in einem schematischen Querschnitt ausschnittweise das Aufnahmeelement des Detektorhalters gemäß FIG 3 mit darauf fixiertem Röntgendetektor, wobei der Röntgendetektor zusammen mit einem oberseitig aufgesetzten Streustrahlenraster fixiert ist,
- FIG 7: in Darstellung gemäß FIG 6 das Aufnahmeelement des Detektorhalters gemäß FIG 3 mit darauf fixiertem Röntgendetektor, wobei der Röntgendetektor ohne Streustrahlenraster eingesetzt ist,
- FIG 8: in Darstellung gemäß FIG 3 eine alternative Ausführung des Detektorhalters sowie den wiederum darauf fixierten Röntgendetektor,
- FIG 9: in Darstellung gemäß FIG 4 den Detektorhalter gemäß FIG 8,
- FIG 10: in Darstellung gemäß FIG 5 den Detektorhalter gemäß FIG 9,
- FIG 11: in Darstellung gemäß FIG 3 eine weitere Ausführung des Detektorhalters mit darauf fixiertem Röntgendetektor,
- FIG 12: in Darstellung gemäß FIG 4 den Detektorhalter gemäß FIG 11,
- FIG 13: in Darstellung gemäß FIG 5 den Detektorhalter gemäß FIG 12 und
- FIG 14: in perspektivischer Ansicht auf die Oberseite den Detektorhalter gemäß FIG 11 beim Einsetzen des Röntgendetektors.

Einander entsprechende Teile sind in allen Figuren stets mit den gleichen Bezugszeichen versehen.

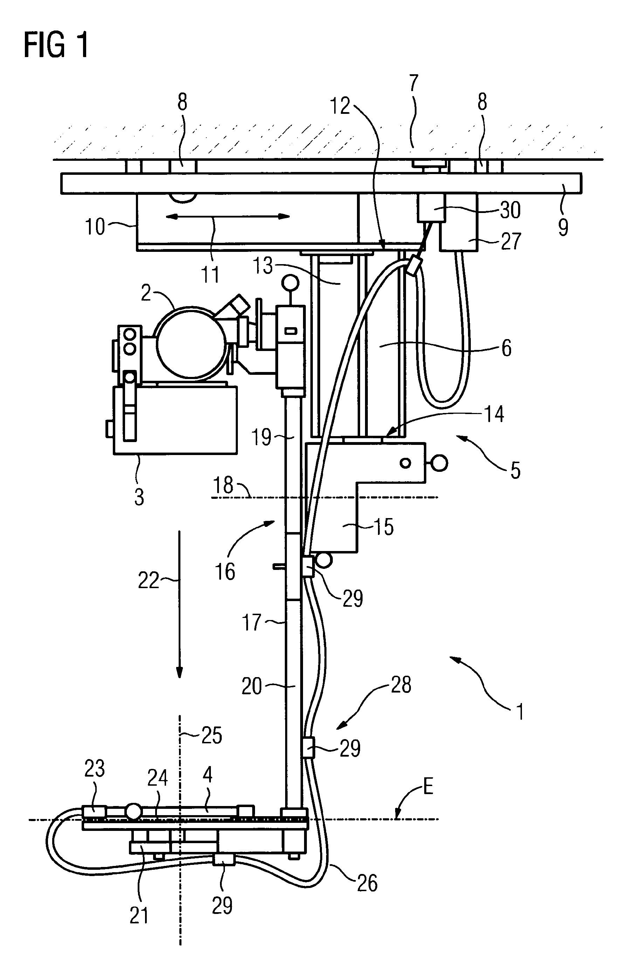

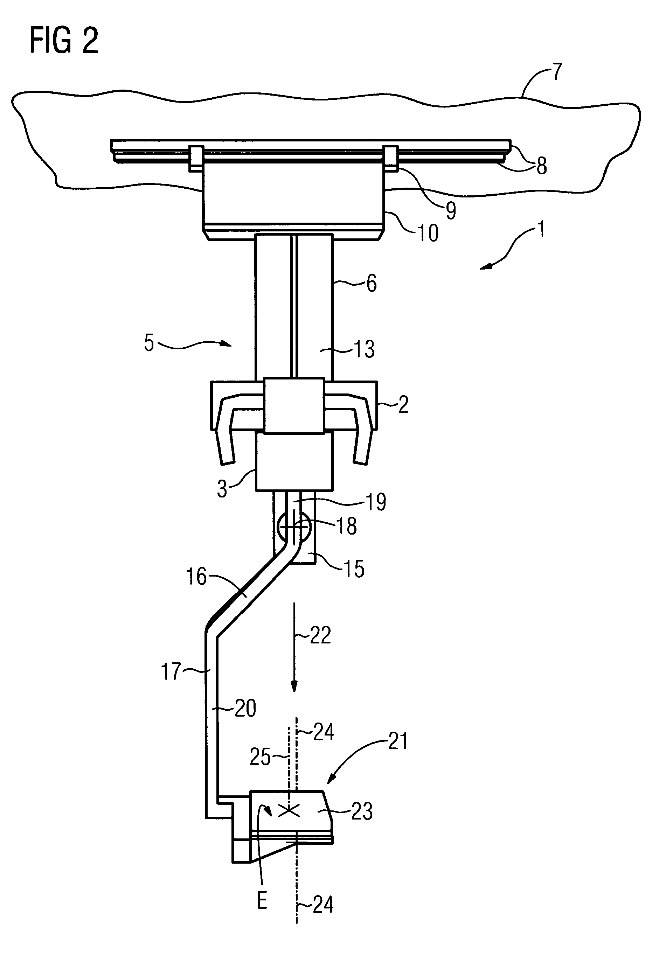

Die in den FIG 1 und 2 schematisch dargestellte Röntgenanlage 1 umfasst einen Röntgenstrahler 2 mit vorgeschalteter Tiefenblende 3 sowie einen als Flachbilddetektor ausgebildeten digitalen Röntgendetektor 4 (in FIG 2 aus Gründen der Vereinfachung nicht dargestellt). Zur Halterung des Röntgenstrahlers 2 und des Röntgendetektors 4 umfasst die Röntgenanlage 1 ein Stativ 5.

Das Stativ 5 ist als Deckenstativ ausgebildet und entsprechend mit einem Sockelgestell 6 an der Raumdecke 7 eines Untersuchungsraumes befestigt. Das Sockelgestell 6 ist hierbei an zwei an der Raumdecke 7 angebrachten Längslaufschienen 8 verschiebbar aufgehängt und kann entlang dieser Längslaufschienen 8 in einer zur Zeichnungsebene der FIG 1 senkrechten Richtung verschoben werden. Im Rahmen des Sockelgestells 6 ist weiterhin eine Querlaufbahn 9 zur Querverschiebung eines Teleskopwagens 10 des Stativs 5 in einer Querrichtung 11 (FIG 1) vorgesehen. Zur Höhenverstellung des Stativs 5 ist an einer Unterseite 12 des Teleskopwagens 10 ein ausziehbarer und nach unten abragender Teleskoparm 13 angeordnet, an dessen unterem Ende 14 ein Tragarm 15 zur Lagerung eines U-Bügels 16 angeordnet ist.

Der U-Bügel 16 umfasst einen langgestreckten Grundholm 17, der um eine Horizontalachse 18 an dem Tragarm 15 drehbar gelagert ist, so dass der Grundholm 17 innerhalb einer (nicht näher dargestellten) vertikalen Schwenkebene verschwenkbar ist. Der Grundholm 17 umfasst einen ersten Schenkel 19 und einen zweiten Schenkel 20, wobei die Schenkel 19 und 20 im Bereich der Horizontalachse 18 aneinander angrenzen und von dort etwa in entgegengesetzter Richtung abstehen. Der Schenkel 19 trägt freiendseitig den Röntgenstrahler 2. Der Schenkel 20 trägt freiendseitig einen Detektorhalter 21, der dazu ausgebildet ist, den Röntgendetektor 4 in einer vorgegebenen Position zu fixieren, in welcher der Röntgendetektor 4 dem Röntgenstrahler 2 - in einer Strahlrichtung 22 der von dem Röntgenstrahler 2 emittierten Röntgenstrahlung gesehen - zentriert gegenübersteht.

Zur Fixierung des Röntgendetektors 4 umfasst der Detektorhalter 21 ein plattenförmiges Aufnahmeelement 23. Um die Detektorposition in Hinblick auf die Erfüllung verschiedener Aufnahmebedingungen flexibel anpassen zu können, kann das Aufnahmeelement 23 (mit dem gegebenenfalls darauf fixierten Röntgendetektor 4) um eine Kippachse 24 gekippt sowie um eine hierzu senkrechte Drehachse 25 gedreht werden.

Die Kippachse 24 ist hierbei parallel zu der Horizontalachse 18 ausgerichtet. Die Drehachse 25 entspricht im Wesentlichen der Flächennormale auf das plattenförmige Aufnahmeelement 23 sowie auf die Detektorfläche des darauf fixierten Röntgendetektors 4, so dass die Drehung des Aufnahmeelements 23 um die Drehachse 25 innerhalb einer durch das Aufnahmeelement 23 bzw. die Detektorfläche des Röntgendetektors 4 aufgespannten Hauptebene E erfolgt und somit die Detektorfläche bei einer Drehung um die Drehachse 25 nicht verkippt wird.

Bei dem Röntgendetektor 4 handelt es sich um einen mobilen Flachbilddetektor, der (in nachfolgend näher beschriebener Weise) an dem Detektorhalter 21 lösbar fixiert ist. Der Röntgendetektor 4 kann auf diese Weise auch separat von dem Stativ 5, insbesondere in einem (nicht explizit dargestellten) Rasterwandgerät oder für eine freie Aufnahme eingesetzt werden.

Der Röntgendetektor 4 ist zur Ansteuerung sowie zur Spannungsversorgung über ein Versorgungskabel 26 (FIG 1) mit einer Steuereinheit 27 verbunden. Die Steuereinheit 27 ist hierbei in Deckennähe, und somit insbesondere über Kopf eines Benutzers, an dem Sockelgestell 6 angebracht. Auf diese Weise ist sichergestellt, dass die Steuereinheit 27 stets zusammen mit der Röntgenanlage 1 verschoben wird. Zum anderen ist die Steuereinheit 27 durch die deckennahe Anordnung effizient aus dem Arbeitsfeld eines Benutzers der Röntgenanlage 1 zurückgezogen und steht hierdurch dem Benutzer bei der Bedienung der Röntgenanlage 1 nicht im Wege.

Um den Benutzer auch durch das Versorgungskabel 26 nicht zu behindern, umfasst die Röntgenanlage 1 des Weiteren eine Kabelführung 28, durch welche das Versorgungskabel 26 außenseitig an dem Stativ 5 entlanggeführt ist. Die Kabelführung 28 umfasst eine Anzahl von als Magnethalterungen ausgebildeten Punkthalterungen 29, mittels welcher das Versorgungskabel 26 lösbar an dem Detektorhalter 21 bzw. dem Grundholm 17 des U-Bügels 16 befestigbar ist. Die Kabelführung 28 umfasst weiterhin eine in Form eines selbsteinziehenden Seilzugs ausgebildete Rückholeinrichtung 30, mittels welcher die für eine Stativaufnahme nicht benötigte Kabellänge des Versorgungskabels 26 automatisch in eine deckennahe Ruhelage zurückgezogen wird (FIG 1). Wird der Röntgendetektor 4 außerhalb des Stativs 5 eingesetzt, so kann das Versorgungskabel 26 unter leichtem Zug gegen die Rückholeinrichtung 30 über seine gesamte Länge ausgezogen werden.

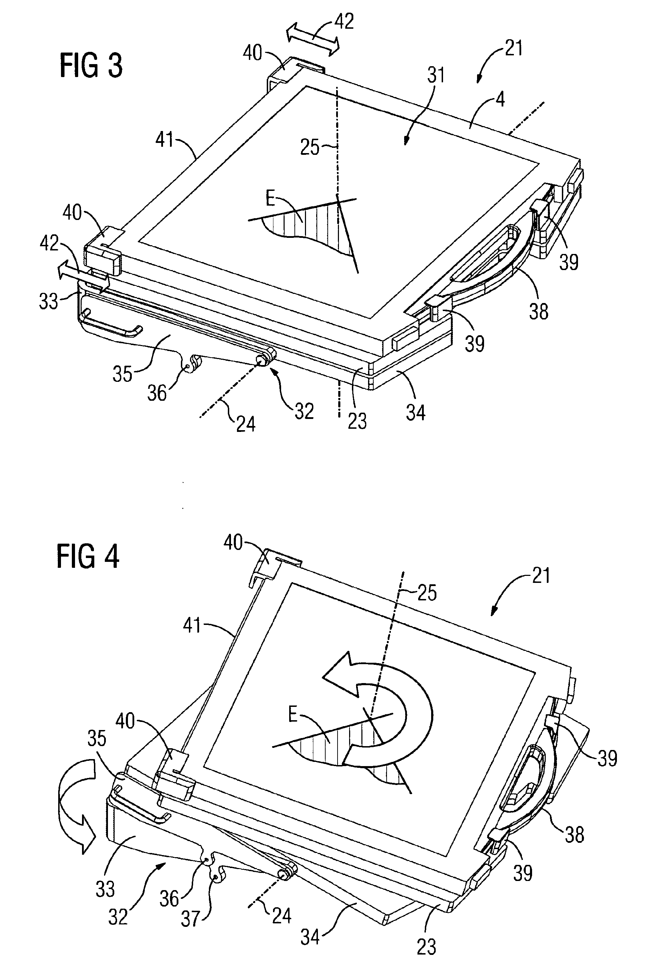

Eine erste Ausführung des Detektorhalters 21 ist in FIG 3 in schräger Draufsicht auf eine dem Röntgenstrahler 2 zugewandte Oberseite 31 dargestellt. FIG 3 sowie die FIG 4 und 5 zeigen den Detektorhalter 21 zusammen mit dem darauf fixierten Röntgendetektor 4. Der Detektorhalter 21 umfasst ein gelenkiges Trägerelement 32, das aus einem U-förmigen Trägerbügel 33 und einer an diesem Trägerbügel 33 um die Kippachse 24 schwenkbar gelagerten Trägerplatte 34 gebildet ist. Auf dieser Trägerplatte 34 liegt wiederum das plattenförmige Aufnahmeelement 23 auf, wobei das Aufnahmeelement 23 mittels einer (nicht näher dargestellten) Bundbuchse um die Drehachse 25 verschwenkbar an der Trägerplatte 34 gelagert ist. Der Trägerbügel 33 ist an dem Schenkel 20 des U-Bügels 16 angeschraubt (FIG 2).

Die Trägerplatte 34 ist bezüglich des Trägerbügels 33 in mehreren Schwenkstellung bezüglich der Verschwenkung um die Kippachse 24 feststellbar, insbesondere 0° (dargestellt in FIG 2), 15° sowie 30° (dargestellt in den FIG 3 und 4). Die Feststellung der Trägerplatte 34 bezüglich des Trägerbügels 33 erfolgt hierbei über einen mit der Trägerplatte 34 entlang der Kippachse 24 drehfest verbundenen Rasthebel 35. In jeder der vorgegebenen Schwenkstellungen wird die Trägerplatte 34 arretiert, indem ein an dem Rasthebel 35 angebrachter Rastbolzen 36 in eine der jeweiligen Schwenkposition entsprechende Rastaufnahme 37 eingreift. Für eine Verstellung der Trägerplatte 34 zwischen verschiedenen Schwenkstellungen wird der Rasthebel 35 an einem freiendseitig angebrachten Handgriff nach außen gezogen, wodurch der Rastbolzen 36 aus der korrespondierenden Rastaufnahme 37 entklinkt, so dass die Trägerplatte 34 gegenüber dem Trägerbügel 33 verschiebbar ist.

Der Verdrehung des Aufnahmeelements 23 gegenüber der Trägerplatte 34 ist eine (nicht näher dargestellte) Gefühlsraste zugeordnet, die das Aufnahmeelement 23 in mehreren Drehstellungen gegen eine ungewollte Selbstverdrehung sichert, wobei diese Sicherung durch Aufbringung eines entsprechenden manuellen Betätigungsdrucks überwindbar ist. Für eine Verdrehung des Aufnahmeelements 23 und des darauf fixierten Röntgendetektors 4 um die Drehachse 25 dient ein an dem Röntgendetektor 4 angebrachter Handgriff 38 als Betätigungselement.

Die Fixierung des Röntgendetektors 4 auf dem Aufnahmeelement 23 erfolgt mittels zweier Klammerelemente 39 sowie zweier weiterer Klammerelemente 40, die den Röntgendetektor 4 durch Umgreifung auf dem Aufnahmeelement 23 verliersicher halten. Die Klammerelemente 39 sind hierbei fest an dem Aufnahmeelement 23 verankert und greifen in Fixierstellung beidseitig des Handgriffs 38 an dem Röntgendetektor 4 an. Die Klammerelemente 40 greifen in Fixierstellung jeweils an den Eckpunkten einer dem Handgriff 38 entgegengesetzten Rückkante 41 des Röntgendetektors 4 an, so dass der Röntgendetektor 4 in Fixierstellung von den Klammerelementen 39 und 40 allseitig umfasst wird. Zur Befestigung bzw. Entnahme des Röntgendetektors 4 sind die Klammerelemente 40 um einen (schematisch angedeuteten) Entriegelungsweg 42 gegen eine Federbelastung aus ihrer Fixierstellung auslenkbar.

Zur Befestigung des Röntgendetektors 4 auf dem Detektorhalter 21 wird der Röntgendetektor 4 mit der Rückkante 41 in die Klammerelemente 40 eingesetzt. Die Klammerelemente 40 werden hierbei durch den Röntgendetektor 4 derart ausgelenkt, dass die griffseitige Kante des Röntgendetektors 4 unter die Klammerelemente 39 einsetzbar ist. Beim Loslassen des Röntgendetektors 4 wird dieser durch die elastisch in ihre Verriegelungsposition zurückkehrenden Klammerelemente 40 automatisch verriegelt.

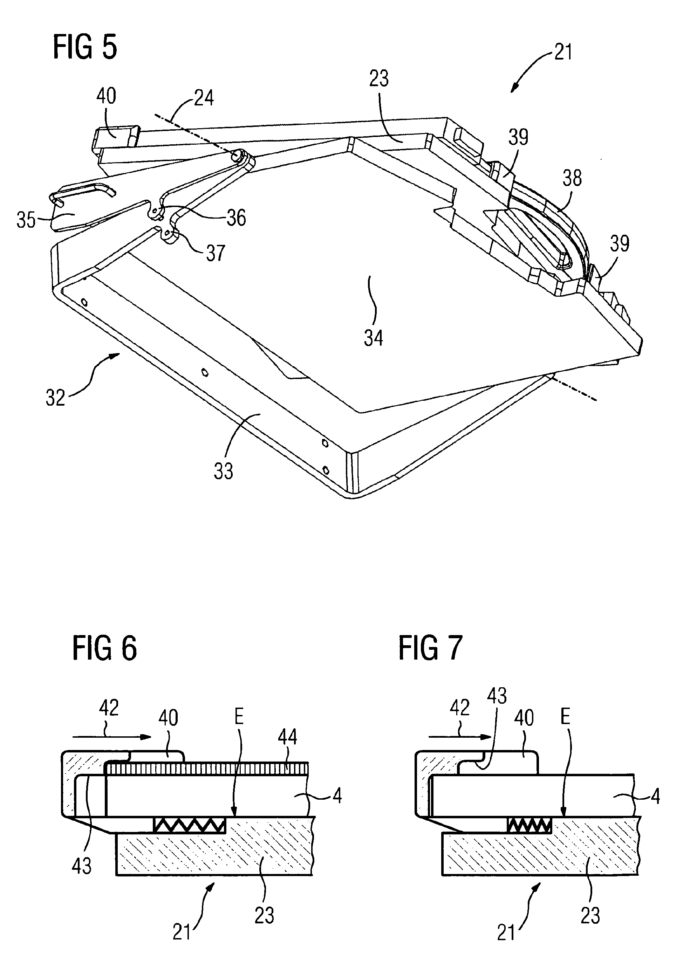

Wie insbesondere aus den FIG 6 und 7 hervorgeht, weisen die Klammerelemente 40 eine abgestufte Innenkontur 43 auf. Dies ist insbesondere sinnvoll, den Röntgendetektor 4 wahlweise mit oder ohne ein oberseitig aufsetzbares Streustrahlenraster 44 sicher, d.h. insbesondere im Wesentlichen spielfrei an dem Detektorhalter 21 haltern zu können.

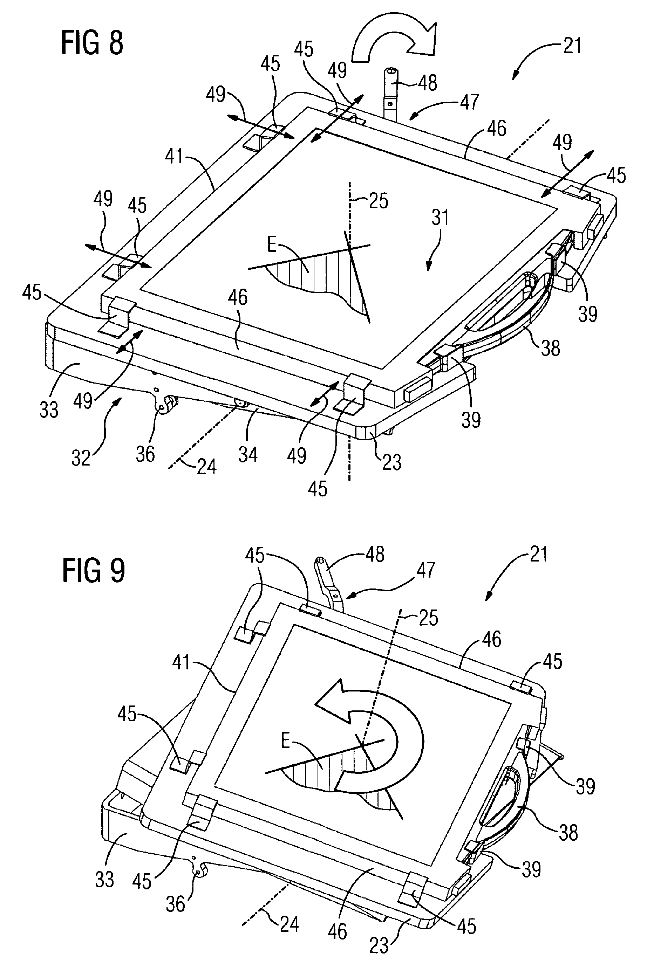

In den FIG 8 bis 10 ist eine weitere Ausführungsform des Detektorhalters 21 mit dem darauf fixierten Röntgendetektor 4 dargestellt. In dieser Ausführungsform sind anstelle der Klammerelemente 40 modifizierte Klammerelemente 45 vorgesehen, die an der Rückkante 41 sowie an gegenüberliegenden Seitenkanten 46 des Röntgendetektors 4 angreifen und diesen zusammen mit den griffseitig angreifenden Klammerelementen 39 in einer Fixierstellung wiederum verliersicher auf dem Aufnahmeelement 23 halten. Zur Entnahme des Röntgendetektors 4 können die Klammerelemente 45 mittels einer Betätigungsmechanik 47, welche über einen Griff 48 betätigbar ist, aus der Fixierstellung entlang eines schematisch dargestellten Entriegelungsweges 49 (FIG 8) in eine zurückgezogene Freigabestellung verschoben werden, in welcher der Röntgendetektor 4 entnommen werden kann. Durch entgegengesetzte Betätigung des Griffes 48 werden die Verriegelungselemente 45 wieder in die Fixierstellung zurückbewegt. Die Klammerelemente 45 sind elastisch flexibel ausgebildet, um den Röntgendetektor 4 sowohl mit als auch ohne Streustrahlenraster 44 spielfrei zu haltern.

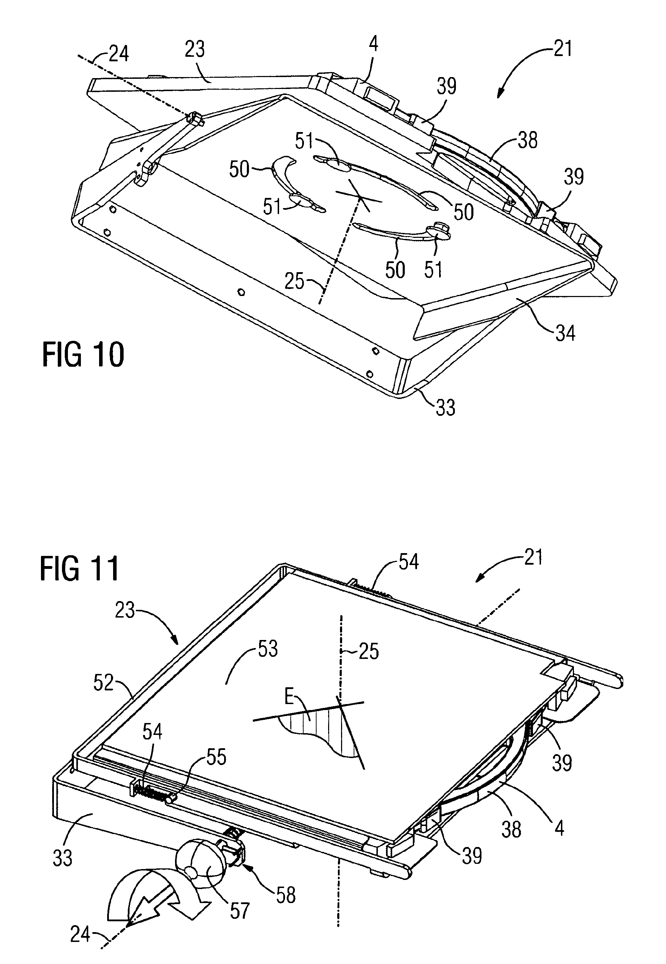

Der zur Verrastung des Aufnahmeelementes 23 in verschiedenen Schwenkpositionen bezüglich des Trägerbügels 33 vorgesehene Rastbolzen 36 ist in der Ausführung des Detektorhalters 21 gemäß den FIG 8 bis 10 als Gefühlsraste ausgebildet, die durch manuelle Verkippung des Aufnahmeelementes 23 überwindbar ist, ohne dass hierfür ein separater Verriegelungsmechanismus betätigt werden müsste. Die schwenkbare Lagerung des Aufnahmeelementes 23 um die Drehachse 25 erfolgt über drei in die Trägerplatte 34 eingebrachte, ringsegmentartige Langlöcher 50 (FIG 10), in denen jeweils ein korrespondierender Fixierbolzen 51 des Aufnahmelementes 23 gleitet.

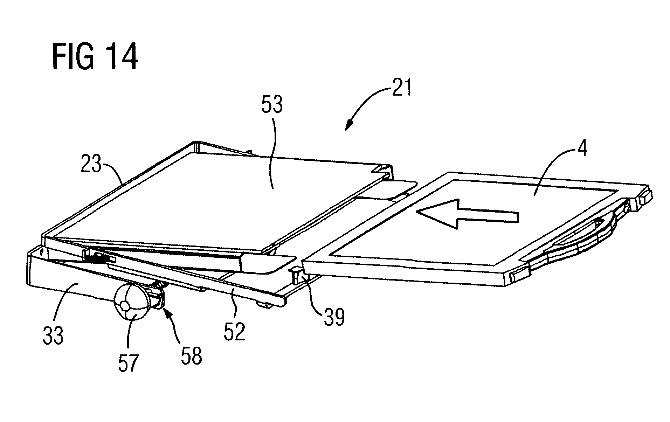

In den FIG 11 bis 14 ist der Detektorhalter 21 in einer weiteren Ausführungsform dargestellt. In dieser Ausführungsform umfasst das Aufnahmelement 23 einen Rahmen 52, in dem eine Detektortasche 53 zur Aufnahme des Röntgendetektors 4 gehaltert ist. Zum Einsetzen des Röntgendetektors 4 in die Detektortasche 53 ist Letztere gegenüber dem Rahmen 52 in eine (in FIG 14 dargestellte) Öffnungsstellung verkippbar. Optional sind hierbei (nicht näher dargestellte) Federelemente vorgesehen, die die Detektortasche 53 in Abwesenheit des Röntgendetektors 4 automatisch in diese Öffnungsstellung drücken, so dass der Röntgendetektor 4 jederzeit einsetzbar ist. Nach dem Einschieben des Röntgendetektors 4 wird die Detektortasche 53 manuell in eine etwa parallel bezüglich des Rahmens 52 ausgerichtete Schließstellung gedrückt. Durch Federelemente 54, die jeweils an einem die Detektortasche 53 an dem Rahmen 52 halternden Führungsbolzen 55 angreifen, wird die Detektortasche 53 zusammen mit dem eingelegten Röntgendetektor 4 gegen die an dem Rahmen 52 fest angebrachten Klammerelemente 39 gedrückt. Durch die den Röntgendetektor 4 in vorstehend beschriebener Weise beidseitig des Handgriffes 38 umgreifenden Klammerelemente 39 wird hierbei die Detektortasche 53 in ihrer Schließstellung gehalten und damit gleichzeitig der Röntgendetektor 4 verliersicher fixiert.

Eine Zentrierung des Röntgendetektors 4 innerhalb der Detektortasche 53 wird durch innerhalb der Detektortasche 53 angeordnete Blattfedern erzielt, die an beiden Seitenkanten 46 sowie optional an einer der Flachseiten des Röntgendetektors 4 angreifen und diesen innerhalb der Detektortasche 53 in eine mittige Position drücken. Durch die Blattfedern wird gleichzeitig sichergestellt, dass der Röntgendetektor 4 mit und ohne Streustrahlenraster 44 sicher gehalten wird.

Zur Verkippung des Aufnahmeelementes 23 um die Kippachse 24 ist in der Ausführung des Detektorhalters 21 gemäß den FIG 11 bis 14 ein Drehgriff 57 vorgesehen. Der Drehgriff 57 wirkt wiederum mit einer in den FIG 11 bis 14 angedeuteten Rasteinrichtung 58 zusammen, welche das Aufnahmeelement 23 und die mit dieser verbundene Trägerplatte 34 in einer von mehreren Schwenkstellung verrastet. Zur Entriegelung der Rasteinrichtung 58 wird der Drehgriff 57 entlang der Kippachse 24 nach außen gezogen, so dass das Aufnahmeelement 23 gegenüber dem Trägerbügel 33 verschwenkt werden kann. Beim Loslassen des Drehgriffs 57 rastet die Rasteinrichtung 58 in der gewählten neuen Schwenkstellung des Aufnahmeelementes 23 ein.

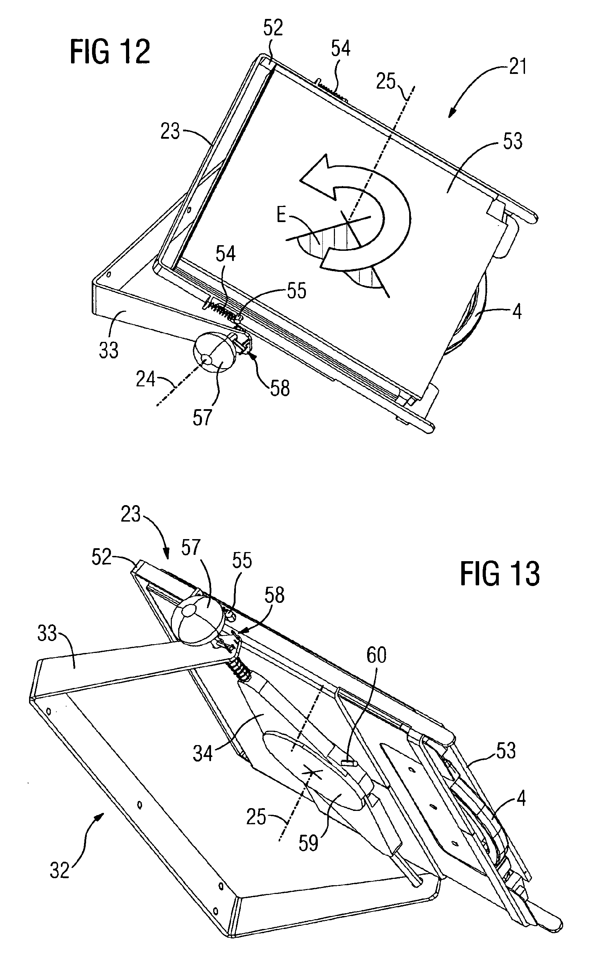

Das Aufnahmeelement 23 und die Trägerplatte 34 sind über ein Drehgelenk 59 verbunden, das die Verschwenkbarkeit des Aufnahmeelements 23 um die Drehachse 25 gewährleistet. Das Drehgelenk 59 ist hierbei mit einem (in FIG 13 erkennbaren) Stift 60 versehen, der als Anschlag die Verdrehung des Aufnahmeelementes 23 gegenüber der Trägerplatte 34 auf einen vorgegebenen Schwenkwinkel begrenzt.

## Patentansprüche

1. Stativ (5) für eine Röntgenanlage (1), mit einem an einem Sockelgestell (6) um eine Horizontalachse (18) drehbar gelagerten U-Bügel (16), der einen ersten Schenkel (19) zur Halterung eines Röntgenstrahlers (2) sowie einen freiendseitig mit einem Detektorhalter (21) versehenen zweiten Schenkel (20) aufweist, wobei der Detektorhalter (21) ein Aufnahmeelement (23) zur verliersicheren Befestigung eines Röntgendetektors (4) in einer vorgegebenen Position umfasst, das gegenüber dem U-Bügel (16) um eine parallel oder tangential bezüglich der Horizontalachse (18) ausgerichtete Kippachse (24) sowie um eine unabhängig von der Kippstellung des Aufnahmeelements (23) bezüglich der Kippachse (24) und einer Hauptebene (E) des Aufnahmeelements (23) senkrechte Drehachse (25) schwenkbar ist, **dadurch gekennzeichnet, dass** der Detektorhalter (21) einen U-förmigen Trägerbügel (33) sowie einen an diesem um die Kippachse (24) schwenkbare Trägerplatte (34) umfasst, an der wiederum das Aufnahmeelement (23) um die Drehachse (25) schwenkbar gelagert ist, wobei des Aufnahmeelement (23) plattenförmig ausgebildet ist und auf der Trägerplatte (34) aufliegt.

2. Stativ nach Anspruch 1,
**dadurch gekennzeichnet, dass** das Aufnahmeelement (23) zur werkzeugfreien Befestigung und Entnahme des Röntgendetektors (4) ausgebildet ist.

3. Stativ (5) nach Anspruch 1 oder 2,
**dadurch gekennzeichnet, dass** das Aufnahmeelement (23) eine Anzahl von Klammerelementen (39,40,45) zur Fixierung des Röntgendetektors (4) aufweist.

4. Stativ (5) nach Anspruch 3,
**dadurch gekennzeichnet, dass** mindestens ein Klammerelement (40) zur Befestigung bzw. Entnahme des Röntgendetektors (4) aus einer Fixierstellung elastisch auslenkbar ist.

5. Stativ (5) nach Anspruch 4,
**dadurch gekennzeichnet, dass** mindestens ein Klammerelement (45) zur Befestigung bzw. Entnahme des Röntgendetektors (4) aus einer Fixierstellung mittels einer Betätigungsmechanik (47) reversibel auslenkbar ist.

6. Stativ (5) nach einem der Ansprüche 1 bis 5,
**dadurch gekennzeichnet, dass** das Aufnahmeelement (23) als Tasche ausgebildet ist, in welche der Röntgendetektor (4) einschiebbar ist.

7. Stativ (5) nach einem der Ansprüche 1 bis 6,
**dadurch gekennzeichnet, dass** der Detektorhalter (21) Rastmittel (36,37,58) umfasst, die das Aufnahmeelement (23) bei einer Schwenkung um die Drehachse (25) und/oder um die Kippachse (24) in einer von mehreren Raststellungen lösbar verrastet.

8. Stativ (5) nach einem der Ansprüche 1 bis 7,
**dadurch gekennzeichnet, dass** das Sockelgestell (6) zur Befestigung an einer Raumwand oder Raumdecke (7) ausgebildet ist.

9. Röntgenanlage (1) mit einem Stativ (5) nach einem der Ansprüche 1 bis 8, mit einem Röntgendetektor (4) sowie mit einer Steuereinheit (27) zur Ansteuerung des Röntgendetektors (4), wobei die Steuereinheit (27) an dem Sockelgestell (6) des Stativs (5) befestigt ist.

10. Röntgenanlage (1) nach Anspruch 9,
**dadurch gekennzeichnet, dass** und wobei die Steuereinheit (27) und der Röntgendetektor (4) durch ein Versorgungskabel (26) verbunden sind, das in einer bezüglich des Stativs (5) außenseitig angeordneten Kabelführung (28) geführt ist.

11. Röntgenanlage (1) nach Anspruch 10,
**dadurch gekennzeichnet, dass** die Kabelführung (28) eine Rückholeinrichtung (30) zur automatischen Rückholung des Versorgungskabels (26) in eine Ruhelage umfasst.

12. Röntgenanlage (1) nach einem der Ansprüche 10 bis 11,
**dadurch gekennzeichnet, dass** die Kabelführung (28) mindestens eine Punkthalterung (29) umfasst, die für eine werkzeugfrei lösbare Fixierung des Versorgungskabels (26) an dem Stativ (5) ausgebildet ist.

## Claims

1. Stand (5) for an X-ray system (1) with a U-frame (16) which is mounted on a base frame (6) so as to be capable of rotating about a horizontal axis (18), which has a first limb (19) for supporting an X-ray emitter (2) and also a second limb (20), which is provided on its free end with a detector support (21), the detector support (21) comprising a recording element (23) which is designed for firm fixing of an X-ray detector (4) in a predetermined position and which can be swivelled in relation to the U-frame (16) about a tilt axis (24) being aligned parallel or tangential to the horizontal axis (18) and also about a rotational axis (25) being perpendicular to the tilt axis (24) and a principal plane (E) of the recording element (23) irrespective of the tilt position of the recording element (23), **characterised in that** the detector support (21) comprises a U-shaped support bracket (33) and a support plate (34) mounted thereon so as to be capable of swivelling about the tilt axis (24), the recording element (23) being mounted in turn on the support plate (34) so as to be capable of swivelling about the rotational axis (25), the recording element (23) being designed as plate-shaped and being supported by the support plate (34).

2. Stand according to claim 1,
**characterised in that** the recording element (23) is designed for fixing and removal of the X-ray detector (4) without tools.

3. Stand (5) according to claim 1 or 2,
**characterised in that** the recording element (23) has a quantity of clamping elements (39, 40, 45) for securing the X-ray detector (4).

4. Stand (5) according to claim 3,
**characterised in that** at least one clamping element (40) can be deflected in an elastic manner from a securing position for the purposes of fixing or removal of the X-ray detector (4).

5. Stand (5) according to claim 4,
**characterised in that** at least one clamping element (45) can be deflected in a reversible manner from a securing position by using an operating mechanism (47) for the purposes of fixing or removal of the X-ray detector (4).

6. Stand (5) according to one of claims 1 to 5,
**characterised in that** the recording element (23) is designed as a holder into which the X-ray detector (4) can be inserted.

7. Stand (5) according to one of claims 1 to 6,
**characterised in that** the detector support (21) comprises latching means (36, 37, 58) which latch the recording element (23), so as to be capable of being released, in one of a plurality of latching positions in the case of a swivelling about the rotational axis (25) and/or about the tilt axis (24).

8. Stand (5) according to one of claims 1 to 7,
**characterised in that** the base frame (6) is designed for fixing to a wall or ceiling (7) of a room.

9. X-ray system (1) with a stand (5) according to one of claims 1 to 8, with an X-ray detector (4) and also with a control unit (27) for controlling the X-ray detector (4), the control unit (27) being fixed on the base frame (6) of the stand (5).

10. X-ray system (1) according to claim 9,
**characterised in that** and wherein the control unit (27) and the X-ray detector (4) are connected by means of a supply cable (26), which is routed in a cable guide (28) arranged on the outside with respect to the stand (5).

11. X-ray system (1) according to claim 10,
**characterised in that** the cable guide (28) comprises a retraction facility (30) for automatic retraction of the supply cable (26) into a rest position.

12. X-ray system (1) according to one of claims 10 to 11,
**characterised in that** the cable guide (28) comprises at least one point support (29), which is designed for a securing of the supply cable (26) on the stand (5) that can be released without tools.

## Revendications

1. Support (5) pour une installation de radiographie (1), avec un étrier en U (16) monté mobile autour d'un axe horizontal (18) sur un châssis formant socle (6) et présentant un premier montant (19) destiné à la fixation d'un émetteur de rayons X (2) ainsi qu'un deuxième montant (20) pourvu, côté de extrémité libre, d'un support de détecteur (21), ledit support de détecteur (21) comprenant un élément de réception (23) destiné à la fixation imperdable d'un détecteur de rayons X (4) dans une position prédéfinie et qui par rapport à l'étrier en U (16), peut pivoter autour d'un axe de basculement (24) orienté parallèlement ou tangentiellement par rapport à l'axe horizontal (18) ainsi qu'autour d'un axe de rotation (25) vertical par rapport à l'axe de basculement (24) et à un plan principal (E) de l'élément de réception (23), indépendamment de la position de basculement de l'élément de réception (23), **caractérisé en ce que** le support de détecteur (21) comprend un étrier support (33) en forme d'U ainsi qu'une plaque support (34) pouvant pivoter autour de l'axe de basculement (24) sur celui-ci et sur laquelle l'élément de réception (23) est quant à lui monté pivotant autour de l'axe de rotation (25), ledit élément de réception (23) étant réalisé sous forme de plaque et étant en appui sur la plaque de support (34).

2. Support selon la revendication 1,
**caractérisé en ce que** l'élément de réception (23) est conçu pour la fixation et le retrait sans outil du détecteur de rayons X (4).

3. Support (5) selon la revendication 1 ou 2,
**caractérisé en ce que** l'élément de réception (23) présente un nombre d'éléments d'attache (39, 40, 45) servant à la fixation du détecteur de rayons X (4).

4. Support (5) selon la revendication 3,
**caractérisé en ce qu'**au moins un élément d'attache (40) servant à la fixation resp. au retrait du détecteur de rayons X (4) peut être sorti de manière élastique de sa position de fixation.

5. Support (5) selon la revendication 4,
**caractérisé en ce qu'**au moins un élément d'attache (45) servant à la fixation resp. au retrait du détecteur de rayons X (4) peut être sorti de manière réversible de sa position de fixation au moyen d'un mécanisme d'actionnement (47).

6. Support (5) selon l'une des revendications 1 à 5,
**caractérisé en ce que** l'élément de réception (23) est conçu sous forme de poche dans laquelle peut se glisser le détecteur de rayons X (4).

7. Support (5) selon l'une des revendications 1 à 6,
**caractérisé en ce que** le support de détecteur (21) comprend des moyens d'encliquetage (36, 37, 58) qui verrouillent de manière amovible l'élément de réception (23) dans une de plusieurs positions d'encliquetage lors d'un pivotement autour de l'axe de rotation (25) et/ou autour de l'axe de basculement (24).

8. Support (5) selon l'une des revendications 1 à 7,
**caractérisé en ce que** le châssis formant socle (6) est conçu pour être fixé au mur d'un local ou au plafond (7) d'un local.

9. Installation de radiographie (1) avec un support (5) selon l'une des revendications 1 à 8, avec un détecteur de rayons X (4) et avec une unité de commande (27) pour commander le détecteur de rayons X (4), ladite unité de commande (27) étant fixée au châssis formant socle (6) du support (5).

10. Installation de radiographie (1) selon la revendication 9,
**caractérisée en ce que**, et dans laquelle, ladite unité de commande (27) et ledit détecteur de rayons X (4) sont reliés par un câble d'alimentation (26), guidé dans un guide-câble (28) disposé du côté extérieur par rapport au support (5).

11. Installation de radiographie (1) selon la revendication 10,
**caractérisée en ce que** ledit guide-câble (28) comprend un dispositif de rappel (30) pour le rappel automatique du câble d'alimentation (26) dans une position de repos.

12. Installation de radiographie (1) selon l'une des revendications 10 à 11,
**caractérisée en ce que** ledit guide-câble (28) comprend au moins une fixation par point (29), conçue pour une fixation amovible sans outil du câble d'alimentation (26) au support (5).
